# EUROPÄISCHE PATENTSCHRIFT

(11) **EP 1 174 079 B1**
(45) Veröffentlichungstag und Bekanntmachung des Hinweises auf die Patenterteilung: **28.09.2005**
(21) Anmeldenummer: 01116990.1
(22) Anmeldetag: 12.07.2001
(51) Int. Cl.: A61B 5/0215, A61N 1/365

(54) **Implantierbare Messvorrichtung, insbesondere Druckmessvorrichtung zur Bestimmung des intrakardialen oder intraluminalen Blutdrucks**
Implantable measuring device especially a pressure measuring device for determination of the intracardiac or intraluminal blood pressure
Dispositif de mesure implantable en particulier un dispositif pour mesurer la pression sanguine intracardiaque ou intraluminale

(30) Priorität: 22.07.2000 DE 10035774; 13.09.2000 DE 10045275
(43) Veröffentlichungstag der Anmeldung: 23.01.2002
(73) Patentinhaber: BIOTRONIK Mess- und Therapiegeräte GmbH & Co Ingenieurbüro Berlin, 12359 Berlin (DE)
(72) Erfinder: Schaldach, Max, Prof. Dr., 91054 Erlangen (DE); Döllgast, Bernhard, 91054 Erlangen (DE); Niewalda, Gregor, 91054 Erlangen (DE)
(74) Vertreter: Hübner, Gerd

(56) Entgegenhaltungen:
- DE-A- 19 624 146
- US-A- 4 191 193
- US-A- 4 509 947
- US-A- 4 698 058
- US-A- 5 174 303

## Beschreibung

Die Erfindung betrifft eine implantierbare Meßvorrichtung, insbesondere Druckmeßvorrichtung zur Bestimmung des intrakardialen oder intraluminalen Blutdrucks, mit den im Oberbegriff des Anspruches 1 angegebenen Merkmalen.

Zum Hintergrund der Erfindung ist am Beispiel einer implantierbaren Druckmeßvorrichtung festzuhalten, daß die Messung des intrakardialen oder intraluminalen - z.B. arteriellen - Blutdrucks in der Herzschrittmachertherapie von Bedeutung ist. Der mittlere arterielle Blutdruck beispielsweise ist ein physiologischer Parameter, mit dessen Hilfe die Stimulationsrate eines ratenadaptiven Herzschrittmachers adaptierbar ist. Der intrakardiale Druckverlauf als weiteres Beispiel für die Anwendung von Druckmessungen kann Aufschluß geben über die Tätigkeit der Herzklappen, den Kontraktionsablauf des Herzens und dessen Kontraktionsstärke. Derartige physiologische Parameter sind im Optimalfall permanent zu überwachen, da sie kontinuierlich als Eingangsgröße bei den verschiedensten Anwendungsfällen benötigt werden können.

Ein dauerhafter Betrieb solcher implantierbarer Druckmeßvorrichtungen, wie z.B. elektromechanischer Druckmeßkatheter, ist bislang praktisch nicht möglich, da die Sensorflächen durch ihre andauernde Beaufschlagung durch den Blutstrom aufgrund der daran stattfindenden Thrombenbildung, Koagulierung und Endothelialisierung mit der Zeit überdeckt werden. Dadurch nimmt ihre Sensitivität stark ab. Dies gilt beispielsweise auch für optisch arbeitende Sensoren, die durch Messung des Amplituden-Verhältnisses zweier Peaks im infraroten Strahlungsband die Blutsauerstoffsättigung ermitteln können, deren Meßeigenschaften durch die erwähnten Überdeckungsprozesse ebenfalls stark verschlechtert werden. Aus den vorstehenden Gründen werden implantierbare Meßsensoren und insbesondere Drucksensoren daher nur temporär eingesetzt.

Aus dem Stand der Technik sind in grundsätzlichem Zusammenhang mit intrakorporal implantierten Kathetern bereits Maßnahmen zur Beseitigung von Verunreinigungen aufgrund des Blutstromes bekannt. So offenbart die US-A-4698058 ein Perfusionskatheter, dessen flüssigkeitsführendes Lumen mittels Ultraschall gereinigt wird. Die Schallwellen werden dabei über ein weiteres, die innere Katheterröhre mit dem zu reinigenden Lumen aufnehmendes zweites Lumen eingestrahlt. Perfusionskatheter werden allerdings üblicherweise nur temporär implantiert. Die sich in ihrem Lumen absetzenden Verunreinigungen beruhen in erster Linie auf unterschiedlichen chemischen oder bluthaltigen Flüssigkeiten, die in beiden Richtungen durch den Katheter geleitet werden.

Die US-A-4 509 947 offenbart die Reinigung einer implantierte Medikamenten-Infusionspumpe mittels Ultraschall. Dabei werden jedoch keine durch den Kontakt der Infusionspumpe mit Körperflüssigkeiten entstandene Ablagerungen entfernt, sondern mögliche Auskristallisierungen des Medikamenten-Reservoirs abgetragen.

Ersichtlich betreffen die Ultraschall-Reinigungsvorrichtungen gemäß den genannten Druckschriften nicht die Problematik der Verunreinigung dauerhaft implantierter Katheter durch Blut. Die Druckschriften stellen somit lediglich technologischen Hintergrund dar.

Aus der US-A-5 174 303 ist ein Herzschrittmaches-Katheter mit einem Grundkörper, einem meßsensitiven Sensor und einer damit gekoppelten Signalleitung bekannt. Reinigungseinrichtung sind an diesem Katheter nicht vorgesehen.

Schließlich offenbart die US-A-4 191 193 einen Katheter mit einem organischen, hochpolymeren, folienähnlichen Piezoelement. Damit können körperintern Druckmessungen durchgeführt werden. Möglichkeiten zur Reingung des Katheters sind auch hier nicht vorgesehen.

Der Erfindung liegt nun die Aufgabe zugrunde, eine implantierbare Meßvorrichtung so auszurüsten, daß sie dauerhaft in einer Körperhöhlung positionierbar ist, die ein zu Ablagerungen auf dem Sensor der Meßvorrichtung führendes Körpermedium leitet oder beinhaltet.

Diese Aufgabe wird durch die Kombination der im Anspruch 1 angegebenen Merkmale gelöst. Demnach ist eine Reinigung des Sensors, der durch ein einheitliches Piezokeramik-Plattenelement gebildet ist, vorgesehen, indem er durch elektrische Anregung über eine Versorgungsleitung in Vibrationen versetzbar ist. Diese übertragen sich auf den Sensor, versetzen diesen in mechanische Schwingungen und entfemen dadurch die Ablagerungen. Der Reinigungsprozeß kann dabei in regelmäßigen Abständen oder bei Bedarf durchgeführt werden. Entsprechend können auch die Frequenz und die Amplitude des Vibrationssignals entweder vorbestimmt oder automatisch nach dem Grad der Verschmutzung oder nach dem Reinigungserfolg optimiert werden. Verschmutzung bzw. Reinigungserfolg sind anhand der elektrischen, optischen oder mechanischen Eigenschaften des Meßsensors ermittelbar.

Ferner sind die Signalleitung und die Versorgungsleitung durch ein einziges gemeinsames Leitungspaar gebildet, das sowohl mit der Meßsignal-Auswerteeinheit für die Druckerfassung als auch mit einer Ansteuerschaltung für die Vibrationsansteuerung des Plattenelementes verbunden ist.

Als bevorzugte Ausführungsform für den Aktuator des Reinigungselementes haben sich flache, quader- oder scheibenförmige Plattenelemente aus Piezokeramik erwiesen, deren Flachseiten zumindest teilweise mit elektrisch leitenden Kontaktflächen für den Anschluß der Versorgungsleitungen belegt sind.

Gemäß bevorzugten Ausführungsformen der Erfindung ist es zur Optimierung der Reinigungswirkung vorgesehen, die Vibrationsfrequenz des Aktuators auf dessen Eigenresonanzfrequenz in Längs- oder Dickenrichtung abzustimmen. Ferner kann der Aktuator mit Mikrokanälen zur Erzeugung von Mikro-Gas-Blasen oder -Flüssigkeits-Strömungen versehen sein. Diese wie Düsen wirkenden Mikrokanäle arbeiten nach der gleichen Funktionsweise wie der Druckkopf eines Tintenstrahldruckers und bedürfen daher keiner detaillierten Erläuterung.

Schließlich kann zur optimierten Energieversorgung der implantierbaren Meßvorrichtung im Hinblick auf eine möglichst unbegrenzte Funktionsdauer die induktive Einkopplung der für die Erzeugung der Vibrationen des Aktuators notwendigen elektrischen Energie über eine Antenne vorgesehen sein. Letztere kann im Implantat, in dessen sogenannten Header, in der Zuleitung zum Sensor oder am Sensor selbst sitzen. Weitere Merkmale, Einzelheiten und Vorteile der Erfindung ergeben sich aus der nachfolgenden Beschreibung, in der ein Ausführungsbeispiel anhand der beigefügten Zeichnungen näher erläutert wird.

Es zeigen:
- Fig. 1: eine schematische perspektivische Darstellung einer dauerhaft zu implantierenden Herzschrittmachers mit einem davon ausgehenden Druckmeßkatheter, und
- Fig. 2: eine schematische perspektivische Darstellung eines Piezokeramik-Plattenelements, wie es als selbstreinigender Drucksensor im Katheter gemäß Fig. 1 zum Einsatz kommt.

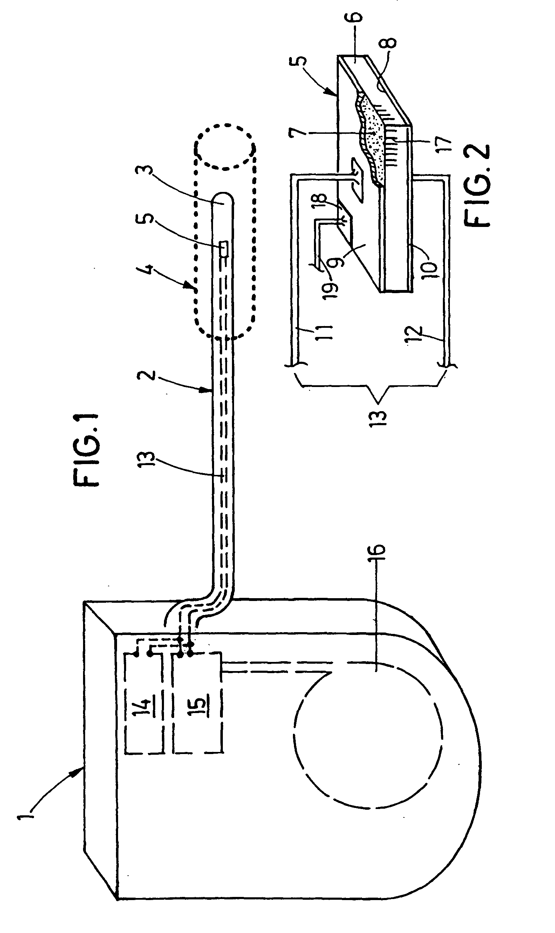

In Fig. 1 ist ein Herzschrittmacher 1 erkennbar, der dauerhaft im Körper eines Patienten implantierbar ist. Er ist vom Typ eines ratenadaptiven Schrittmachers, dessen Stimulationsrate über den mittleren arteriellen Blutdruck als physiologischer Parameter eingestellt wird. Zu dessen Ermittlung ist mit dem Herzschrittmacher 1 ein Katheter 2 gekoppelt, dessen distales Ende 3 in der punktiert angedeuteten Arterie 4 positionierbar ist. Am distalen Ende 3 des Katheters 2 ist ein selbstreinigender Drucksensor 5 angeordnet, der den in der Arterie 4 herrschenden Druck erfaßt.

Wie aus Fig. 2 deutlich wird, besteht dieser Drucksensor 5 aus einem flachquaderförmigen Piezokeramik-Plattenelement 6, dessen beiden Flachseiten 7, 8 mit einer Kontaktschicht 9, 10 beispielsweise aus Silber belegt sind. An diese Kontaktschichten 9, 10 sind jeweils ein Leitungsdraht 11, 12 eines Leitungspaars 13 (Fig. 1) durch Löten ankontaktiert, das einerseits mit einer Meßsignalauswerteeinheit 14 und andererseits einer Ansteuerschaltung 15 zur Erzeugung einer hochfrequenten Wechselspannung zwischen den beiden Leitungsdrähten 11, 12 verbunden ist.

Das Piezo-Plattenelement 6 dient - wie erwähnt - einerseits als Drucksensor, da der auf das Plattenelement ausgeübte Druck zu einer repräsentativen Spannung zwischen den beiden Kontaktschichten 9, 10 führt, die von der Meßsignal-Auswerteeinheit 14 erfaßbar und in einen entsprechenden Druckwert umsetzbar ist. Gleichzeitig dient das Piezokeramik-Plattenelement 6 auch als Reinigungselement in Form eines piezokeramischen Aktuators, der durch die von der Ansteuerschaltung 15 zur Verfügung gestellte, hochfrequente Wechselspannung in Vibrationen versetzt wird, die zu einer Entfernung von Ablagerungen auf dem Plattenelement 6 sorgen. Die Energie für diese hochfrequente Wechselspannung wird induktiv von außen auf den Herzschrittmacher 1 durch ein elektromagnetisches Wechselfeld übertragen, das von der mit der Ansteuerschaltung 15 gekoppelten Antenne 16 im Herzschrittmacher 1 empfangen wird. Die Frequenz der Wechselspannung liegt im Bereich der Eigenfrequenz des Piezokeramik-Plattenelementes 6 und damit in der Größenordnung von kHz. Durch eine Anpassung der Anregungsfrequenz an die Eigenresonanzfrequenz des Plattenelementes 6 ist eine besonders intensive Schwingungsanregung des Plattenelementes 6 möglich. Zur einfacheren Ansteuerung und adaptiven Nachführung der Schwingungsanregung kann ein Teil der Kontaktschicht 9, 10 als Rückkoppelungselektrode 18 ausgebildet sein, deren Signal über eine zusätzliche Leitung 19 der Ansteuerschaltung 15 zugeführt wird (Fig. 2). Dies führt ebenso zu einer Optimierung des Reinigungseffektes, wie die Auslegung des Plattenelementes 6 als Mehrschichtaufbau.

Zur weiteren Verbesserung kann das Piezokeramik-Plattenelement 6 - wie in Fig. 2 angedeutet - mit Mikrokanälen 17 versehen sein, die bei einer Schwingungsanregung des Plattenelementes 6 zusätzlich für ein impulsartiges Ausstoßen von Gasblasen oder Flüssigkeits-Jets sorgen. Dies wirkt auf Anlagerungen auf dem Plattenelement 6 weiter stark ablösend.

Vorversuche mit piezokeramischen Aktuatoren in Form von Plattenelementen mit einer Fläche von 5 mm x 5 mm und Dicken von 0,2 mm, 0,5 mm und 1 mm haben bei zwei unterschiedlichen Keramikmaterialien abgeschätzte planare oder längsgerichtete Eigenresonanzfrequenzen zwischen etwa 10 kHz und 15 kHz ergeben. Diese mit vollflächigen Silberelektroden und angelöteten Kupferlackdraht versehenen Plattenelemente wurden für jeweils 15 Minuten, 1 Stunde, 24 Stunden und 4 Tagen in Rinderblut bei 37°C gelagert. Es bildeten sich dadurch je nach Lagerdauer und Materialart Ablagerungen in Form von Koagulationsschichten bis hin zu Fibrin- und Thrombenbildung auf den Plättchen.

Zur Selbstreinigung wurden anschließend diese Plättchen mit einer Sinusspannung im Bereich der oben angegebenen Frequenzen und einer Amplitude von 5 Volt bis 15 Volt angesteuert. Im Ergebnis betrug die Vibrationsdauer, mit der unter den geschilderten Umständen eine effektive Reinigung erzielt werden konnte, zwischen 30 Sekunden und 300 Sekunden je nach Art des Piezoelementes und dessen Lagerdauer. Die Elemente mit einer Schichtdicke von 0,2 mm wiesen dabei gegenüber den dickeren Elementen bessere Eigenschaften, nämlich in erster Linie kürzere Reinigungsdauern auf, was an der aufgrund der geringeren Dicke höheren Feldstärke liegen dürfte.

Bei den oben erwähnten, für die Vorversuche ausgewählten piezokeramischen Aktuatoren liegen im übrigen die Dickeneigenfrequenzen im Bereich von etwa 2 MHz bis 10 MHz. Bei einer Anregung des Aktuators in diesem Frequenzbereich sind zusätzlich Kavitationseffekte zu erwarten, die zu einer intensiven Reinigung der Aktuatoren beitragen.

## Patentansprüche

1. Implantierbare Meßvorrichtung, insbesondere Druckmeßvorrichtung zur Bestimmung des intrakardialen oder intraluminalen Blutdruckes, umfassend
- einen in der jeweiligen Körperhöhlung (4) positionierbaren, vorzugsweise katheterartigen Grundkörper (2),
- einen meßsensitiven Drucksensor (5) am Grundkörper (2) in Form eines einheitlichen Piezokeramik-Plattenelements (6),
- eine Meßsignal-Auswerteeinheit (14),
- eine mit dem Drucksensor (5) gekoppelte Signalleitung (13) zur Übertragung der dem zu erfassenden Meßwert entsprechenden, vom Drucksensor (5) generierten Meßsignale zu der Meßsignal-Auswerteeinheit (14),
**gekennzeichnet durch**
- eine Ansteuerschaltung (15) für die Vibrationsansteuerung des Plattenelements (6),
- eine Versorgungsleitung (13) zwischen Ansteuerschaltung (15) und Plattenelement (6), über die das Plattenelement (6) zur Entfernung von Ablagerungen **durch** elektrische Anregung in Vibrationen versetzbar ist,
- wobei die Signal- und Versorgungsleitungen **durch** ein gemeinsames Leitungspaar (13) gebildet sind, das mit der Meßsignal-Auswerteeinheit (14) und der Ansteuerschaltung (15) verbunden ist.

2. Meßvorrichtung nach Anspruch 1, **dadurch gekennzeichnet, daß** der Aktuator ein flaches Plattenelement (6) aus Piezokeramik ist, dessen Flachseiten (7, 8) zumindest teilweise mit elektrisch leitenden Kontaktflächen (9, 10) für die Versorgungsleitungen (13) belegt sind.

3. Meßvorrichtung nach Anspruch 2, **dadurch gekennzeichnet, daß** an mindestens einer Kontaktfläche (9) eine mit einer Ansteuerschaltung (15) für die Vibrationsansteuerung des Plattenelementes (6) verbundene Rückkoppelungselektrode (18) abgeteilt ist.

4. Meßvorrichtung nach einem der Ansprüche 1 bis 6, **dadurch gekennzeichnet, daß** die Vibrationsfrequenz des Plattenelements (6) dessen Eigenresonanzfrequenz entspricht.

5. Meßvorichtung nach einem der Ansprüche 1 bis 4, **dadurch gekennzeichnet, daß** das Plattenelement (6) mit Mikrokanälen (17) zur Erzeugung von Mikro-Gas- oder -Flüssigkeits-Strömungen versehen ist.

6. Meßvorrichtung nach einem der Ansprüche 1 bis 5, **dadurch gekennzeichnet, daß** die für die Erzeugung der Vibrationen des Plattenelements (6) notwendige elektrische Energie über eine Antenne (16) induktiv einkoppelbar ist.

## Claims

1. An implantable measuring device, particularly a pressure measuring device for determining the intracardial or intraluminal blood pressure, comprising
- a preferably catheter-like main body (2) that can be positioned in the given body cavity (4),
- a measurement-sensitive pressure sensor (5) on the main body (2) in the form of a uniform piezoceramic plate element,
- a measuring signal evaluation unit (14),
- a signal line (13) coupled to the pressure sensor (5) for transmitting to the measuring signal evaluation unit (14) the measuring signals generated by the pressure sensor (5) corresponding to the detected measuring value,
**characterized by**
- a control circuit (15) for the vibration actuation of the plate element (6),
- a supply line (13) between control circuit (15) and plate element (6), via which the plate element (6) can be set in vibrations by electrical excitation for the removal of deposits,
- wherein the signal line and the supply line are formed by a common pair of leads (13) that is connected to the measuring signal evaluation unit (14) and to the control circuit (15).

2. A measuring device according to claim 1, **characterized in that** the actuator is a thin plate element (6) of piezoceramics, the large faces (7, 8) of which are at least partially covered with electrically conducting contact areas (9, 10) for the supply lines (13).

3. A measuring device according to claim 2, **characterized in that** a feedback electrode (18) that is connected to a control circuit (15) for the vibration actuation of the plate element (6) is partitioned off on at least one contact area (9).

4. A measuring device according to any of claims 1 through 3, **characterized in that** the vibration frequency of the actuator (6) corresponds to its self-resonant frequency.

5. A measuring device according to any of claims 1 through 4, **characterized in that** the plate element (6) is provided with microchannels (17) for generating micro flows of gas or fluid.

6. A measuring device according to any of claims 1 through 5, **characterized in that** the energy required to generate the vibrations of the actuator (6) can be coupled-in inductively via an antenna (16).

## Revendications

1. Dispositif de mesure implantable, notamment dispositif de mesure de pression, destiné à la mesure de la pression sanguine intracardiaque ou intraluminale, comportant
- un corps de base (2), de préférence de type cathéter, pouvant être positionné dans la cavité corporelle (4) respective,
- sur le corps de base (2), un détecteur de pression (5) à mesure sensitive sous la forme d'un élément à plaque (6) homogène en piézocéramique,
- une unité d'évaluation (14) des signaux de mesure,
- une ligne de signalisation (13) couplée au détecteur de pression (5) pour la transmission à l'unité d'évaluation (14) des signaux de mesure générés par le détecteur de pression (5) correspondant à la valeur de mesure à saisir,
**caractérisé par**
- un circuit de commande (15) pour la commande de vibrations de l'élément à plaque (6),
- une ligne d'alimentation (13) entre le circuit de commande (15) et l'élément à plaque (6), par l'intermédiaire de laquelle l'élément à plaque (6) peut être mis en vibrations par une excitation électrique pour l'élimination de dépôts,
- les lignes de signalisation et d'alimentation étant constituées d'un couple de lignes (13) communes, qui est relié à l'unité d'évaluation (14) des signaux de mesure et au circuit de commande (15).

2. Dispositif de mesure selon la revendication 1, **caractérisé en ce que** l'actionneur est un élément à plaque (6) plat en piézocéramique, dont les faces plates (7, 8) sont dotées, au moins partiellement, de surfaces de contact (9, 10) électriquement conductrices pour les lignes d'alimentation (13).

3. Dispositif de mesure selon la revendication 2, **caractérisé en ce qu'**une électrode de rétroaction (18) reliée à un circuit de commande (15) pour la commande de vibrations de l'élément à plaque (6) est prévue sur au moins une surface de contact (9).

4. Dispositif de mesure selon l'une des revendications 1 à 3, **caractérisé en ce que** la fréquence des vibrations de l'élément à plaque (6) correspond à sa propre fréquence de résonance.

5. Dispositif de mesure selon l'une des revendications 1 à 4, **caractérisé en ce que** l'élément à plaque (6) est pourvu de micro-canaux (17) destinés à engendrer des écoulements de micro-gaz ou de liquides.

6. Dispositif de mesure selon l'une des revendications 1 à 5, **caractérisé en ce que** l'énergie électrique nécessaire à la génération des vibrations de l'élément à plaque (6) peut être couplée par induction par l'intermédiaire d'une antenne (16).
